# EUROPÄISCHE PATENTSCHRIFT

(11) **EP 1 356 769 B1**
(45) Veröffentlichungstag und Bekanntmachung des Hinweises auf die Patenterteilung: **23.11.2005**
(21) Anmeldenummer: 03005739.2
(22) Anmeldetag: 14.03.2003
(51) Int. Cl.: A61B 6/00

(54) **Röntgendiagnostikgerät für Mammographieuntersuchungen**
X-ray diagnostic apparatus for mammographies
Appareil de dignostique à rayons X, pour mammographie

(30) Priorität: 23.04.2002 SE 0201211
(43) Veröffentlichungstag der Anmeldung: 29.10.2003
(73) Patentinhaber: SIEMENS AKTIENGESELLSCHAFT, 80333 München (DE)
(72) Erfinder: Andreasson, Jesper, 177 40 Järfälla (SE); Karlsson, Stefan, 191 34 Sollentuna (SE); Teksöz-Wibrink, Josefin, SE/S-Bromma (SE)

(56) Entgegenhaltungen:
- EP-A- 0 775 467
- EP-B- 0 324 358
- EP-B- 0 370 089
- US-A- 5 170 420

## Beschreibung

Die Erfindung betrifft ein Röntgendiagnostikgerät für Mammographieuntersuchungen mit einem Arm für eine Röntgenröhre und einem Objekttisch sowie einer zwischen der Röntgenröhre und dem Objekttisch angeordneten Kompressionsplatte, die mit dem Arm verbunden und entlang diesem verschiebbar ist, wobei der Arm um eine horizontale Welle drehbar und entlang einer fiktiven Längsachse eines Stativs in der Höhe verschiebbar ist, wobei der Arm mittels eines ersten Mittels um die horizontale Welle drehbar ist, dass die Welle an einer Anordnung befestigt ist, die mittels eines zweiten Mittels seitlich verschiebbar ist, wodurch der Arm seitlich verschiebbar ist, dass die Anordnung zum seitlichen Verschieben des Armes an einer mit dem Stativ verbundenen Hubanordnung verschiebbar angebracht ist, die mittels eines dritten Mittels in der Höhe verschiebbar ist und dass eine Steuervorrichtung vorhanden ist, die mittels eines Steuerprogramms, das Ausgangssignale von Lagesensoren, die die Höhen- und Seitenlagen des Armes sowie von einem Drehmelder, der den Drehwinkel des Armes angibt, entgegennimmt, das zweite und das dritte Mittel für die Verschiebung des Armes seitlich und in die Höhe sowie das erste Mittel für die Drehung des Armes derart steuert, dass eine Drehung des Armes um eine entlang der fiktiven Längsachse des Stativs beliebig wählbare, fiktive Achse ermöglicht wird.

Ein Röntgendiagnostikgerät dieser Art ist durch die europäische Patentschrift EP 0 324 358 B1 bekannt. Die Röntgenröhre und der Objekttisch, die zusammen einen Durchleuchtungskopf bilden, weisen zueinander einen konstanten Abstand auf. Der Durchleuchtungskopf ist als Ganzes in seiner Höhe verstellbar. Aufgrund des konstruktiven Aufbaus müssen die meisten Patienten beim Wechsel von einer ersten vertikalen Aufnahmestellung in eine seitliche Aufnahmestellung eine gewisse seitliche Lagekorrektur vornehmen.

Die ersten und zweiten Mittel können bei dem Röntgendiagnostikgerät gemäß der europäischen Patentschrift EP 0 324 358 B1 als leistungsschwache und damit kleine und billige Motoren ausgebildet sein, die mit Hilfe der Steuervorrichtung den Arm um die erwähnte fiktive wählbare Achse drehen können. Durch den kompakten Aufbau der Armanordnung ist auch keine Gewichtsausgleichsvorrichtung für die Drehbewegungen des Armes erforderlich, da diese Drehbewegungen immer in der Nähe des Schwerpunktes des Armes erfolgen.

Ein weiteres Röntgendiagnostikgerät ist in der europäischen Patentschrift EP 0 370 089 B1 beschrieben. Bei diesem Gerät liegt die Drehachse des Armes außerhalb des Schwerpunktes des Armes. Damit der Arm mit der Röntgenröhre, der Kompressionsplatte und dem Objekttisch um die erwähnte Drehachse gedreht werden kann, ist das freie Ende der Drehachse mit einem mit dem Ende fest verbundenen Zahnrad versehen, das mit Hilfe eines verhältnismäßig starken und damit verhältnismäßig großen und teueren Motors angetrieben wird. Eine Gasfedervorrichtung dient als ein zusätzlicher Gewichtsausgleich des Armes. Die vertikale Verschiebung des Armes erfolgt mit Hilfe eines Motors und einer Zahnstange, die von der Drehvorrichtung des Armes getrennt angeordnet sind.

Bei einer Routineuntersuchung einer Brust eines Patienten werden eine erste vertikale Aufnahme und danach eine zweite Aufnahme vorgenommen, bei der der Arm zwischen 45° und 60° gedreht worden ist. In Verbindung mit einer weiteren Untersuchung kann auch eine Aufnahme gemacht werden, wenn der Arm etwa 90° gedreht worden ist. Um zu vermeiden, dass der Patient beim Drehen des Armes von einer ersten in eine zweite Aufnahmestellung einen Schritt zur Seite machen muss, ist die Verlängerung der fiktiven Zentrumsachse der Drehachse beim Gegenstand des genannten europäischen Patents so angeordnet, dass sie unmittelbar oberhalb des Objekttisches liegt. Der Abstand zwischen dem Objekttisch und der erwähnten Zentrumsachse ist auch in diesem Fall fest, was bedeutet, dass die meisten Patienten beim Wechsel von einer ersten vertikalen Aufnahmestellung in einer seitlichen Aufnahmestellung eine gewisse seitliche Lagekorrektur vornehmen müssen. Lediglich wenige Patienten haben für dieses Gerät eine ideale Bruststärke, so dass die erwähnte fiktive Zentrumsachse mit der fiktiven Zentrumsachse der komprimierten Brust achsfluchtend liegt.

Der Erfindung liegt die Aufgabe zugrunde, ein Röntgendiagnostikgerät für Mammographieuntersuchungen der eingangs genannten Art zu schaffen, bei dem der Arm von einer Aufnahmestellung in eine andere Aufnahmestellung mit einem gewünschten Drehwinkel um ein für den Patienten angepasstes Isozentrum gedreht werden kann, wobei der Patient unabhängig von der Brustgröße seine Position beibehalten kann.

Diese Aufgabe wird bei einem Röntgendiagnostikgerät gemäß dem Oberbegriff des Anspruchs 1 dadurch gelöst, dass die Steuervorrichtung einen Abstandsmesser aufweist, der an der Kompressionsplatte angeordnet ist und durch den der Abstand der Kompressionsplatte vom Objekttisch messbar ist, so dass die fiktive horizontale Achse achsfluchtend mit der fiktiven Zentrumsachse einer zu untersuchenden Brust gelegt ist, wenn sich diese in einer Kompressionslage befindet.

Dadurch, dass die Steuervorrichtung individuell für jeden Patienten eine optimale fiktive Achse ermittelt, um die der Arm gedreht werden kann, ist unabhängig von der Größe der Brust eine Umpositionierung des Patienten von einer vertikalen Aufnahmestellung in eine gewünschte seitliche Aufnahmestellung nicht notwendig. Die fiktive horizontale Achse ist immer entlang der fiktiven Längsachse des Stativs angeordnet.

In einer vorteilhaften Weiterbildung des Röntgendiagnostikgerätes nach der Erfindung wird vorgeschlagen, dass die fiktive Achse mit einem Abstand vom Objekttisch liegt, der dem halben Abstand zwischen dem Objekttisch und der Kompressionsplatte entspricht, wenn die Kompressionsplatte sich in einer Kompressionslage befindet. Durch die Wahl, den Arm um die beschriebene fiktive Achse zu drehen ist sichergestellt, dass der Patient, unabhängig von der Bruststärke, beim Wechseln der Aufnahmewinkel nicht umpositioniert werden muss.

Die Erfindung ist nachfolgend anhand eines in den Zeichnungen dargestellten Ausführungsbeispiels näher erläutert. Es zeigen:
- FIG. 1: eine Seitenansicht eines Röntgendiagnostikgeräts mit einer Armanordnung nach der Erfindung,
- FIG. 2: eine perspektivische Ansicht einer Armanordnung nach FIG. 1,
- FIG. 3: eine perspektivische Ansicht einer Armanordnung nach FIG. 1 und 2, bei der der Arm in einer weiteren Aufnahmestellung als der, die in der FIG. 2 dargestellt ist, gezeigt ist und
- FIG. 4: eine Steuervorrichtung der Armanordnung in einem schematisch dargestellten Blockschaltbild.

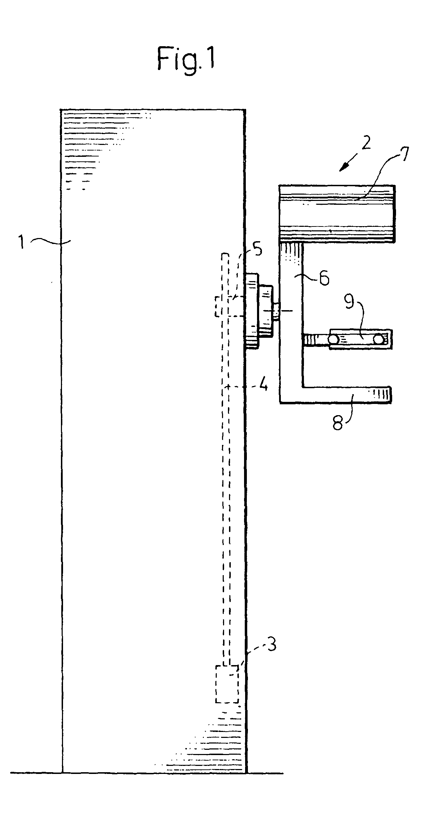

In der FIG. 1 ist ein Röntgendiagnostikgerät für Mammographieuntersuchungen, d.h. ein Mammographiegerät in schematischer Form gezeigt mit einem Stativ 1, das eine später näher beschriebene Armanordnung 2 trägt. Die Armanordnung 2 ist mit Hilfe eines Motors 3 und einer Schraube 4, deren eines Ende mit einem an der Armvorrichtung fest angeordneten Teil 5 verbunden ist, in der Höhe verschiebbar.

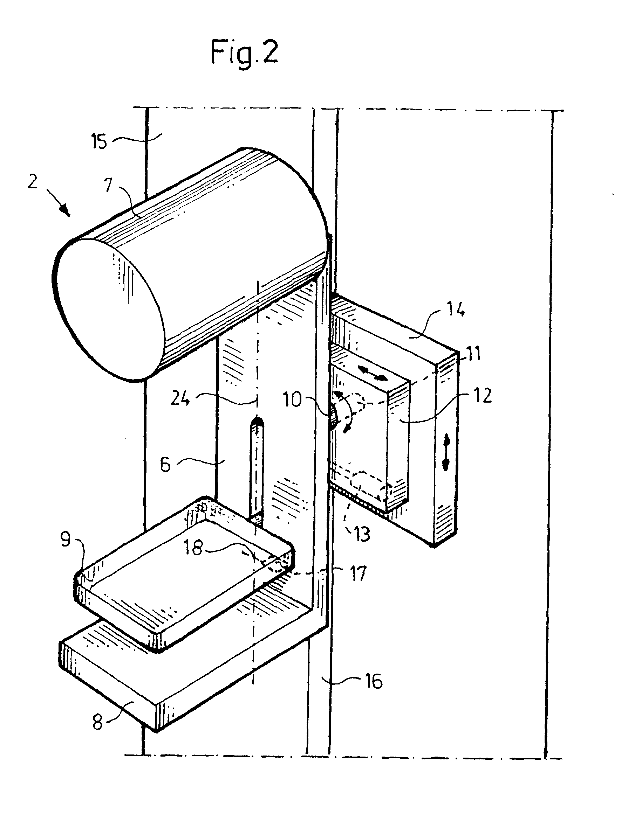

In der FIG. 2 ist die Armanordnung 2 gezeigt, bestehend aus einem Arm 6, einer am Arm 6 angeordneten Röntgenröhre 7 und einem Objekttisch 8 sowie einer zwischen der Röntgenröhre 7 und dem Objekttisch 8 angeordneten Kompressionsplatte 9, die mit dem Arm 6 verbunden und entlang diesem verschiebbar ist. Hier ist auch gezeigt, dass der Arm 6 der Armanordnung 2 mit Hilfe eines Motors 11 um eine horizontale Welle 10 drehbar ist, wobei die Welle 10 an einem Seitenwagen 12 befestigt ist, d.h. einem Wagen, der mit Hilfe eines Motors 13 seitlich verschiebbar ist, wodurch auch der Arm 6 seitlich verschiebbar ist. Der Wagen 12 ist an einem Hubwagen 14 verschiebbar angeordnet, der über das bereits erwähnte Teil 5 mit der Schraube 4 zum Verschieben des Hubwagens 14 und damit zum Verschieben der Armanordnung 2 in der Höhe verbunden ist. In dieser Figur ist auch die Stirnseite 15 des Mammographiegerätes gezeigt. Diese Stirnseite 15 ist mit einem Schlitz 16 versehen, in dem das Teil 5 verläuft und entlang dem daher die Armvorrichtung 2 in der Höhe verschiebbar ist.

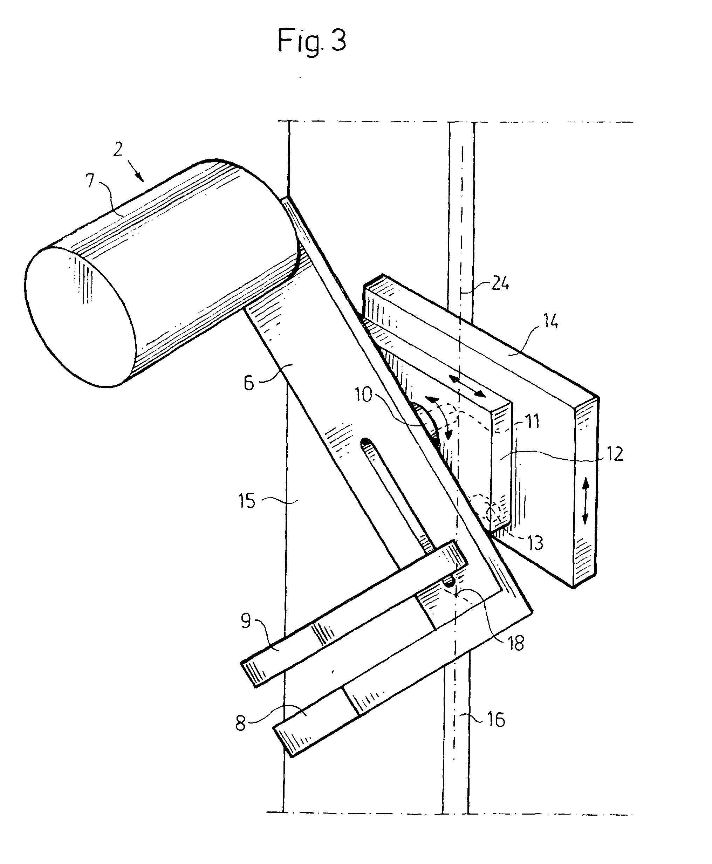

Vor einer Untersuchung einer Brust eines in der Figur nicht dargestellten Patienten wird die Armanordnung 2 entlang einer fiktiven Längsachse 24 des Stativs 1 in eine Lage verschoben, in der der Objekttisch 8 eine für den Patienten passende Lage einnimmt. Die fiktive Längsachse 24 verläuft vorzugsweise parallel mit dem Schlitz 16. Mit Hilfe der Kompressionsplatte 9 wird nun die Brust gegen den Objekttisch 8 gedrückt, bis eine optimale Kompression der Brust für eine Aufnahme erhalten worden ist, wobei eine vertikale Aufnahme der Brust ausgeführt wird. Die Kompressionsplatte 9 ist erfindungsgemäß mit einem Abstandsmesser 17 versehen, der in dieser Lage den Abstand zum Objekttisch 8 misst. Der Abstandsmesser 17, der vorzugsweise an dem Ende der Kompressionsplatte 9, das gegen den Arm 6 gerichtet ist, angebracht ist, wird hier später beschrieben. Nach einer ersten vertikalen Aufnahme der Brust wird der Patient aus der komprimierten Lage gelöst, wobei der Arm 6 von einer beschriebenen vertikalen Aufnahmestellung in eine zweite Aufnahmestellung mit einem Winkel zur ersten Aufnahmestellung von 45° bis 60° gedreht wird. Eine solche zweite Aufnahmestellung ist in der FIG. 3 gezeigt. Der Arm 6 wird nun um eine ideale fiktive Achse 18 gedreht, die mit einem Abstand vom Objekttisch 8 liegt, der dem halben Abstand zwischen dem Objekttisch 8 und der Kompressionsplatte 9 entspricht, wenn sich die Kompressionsplatte 9 in ihrer bereits beschriebenen Kompressionslage befindet.

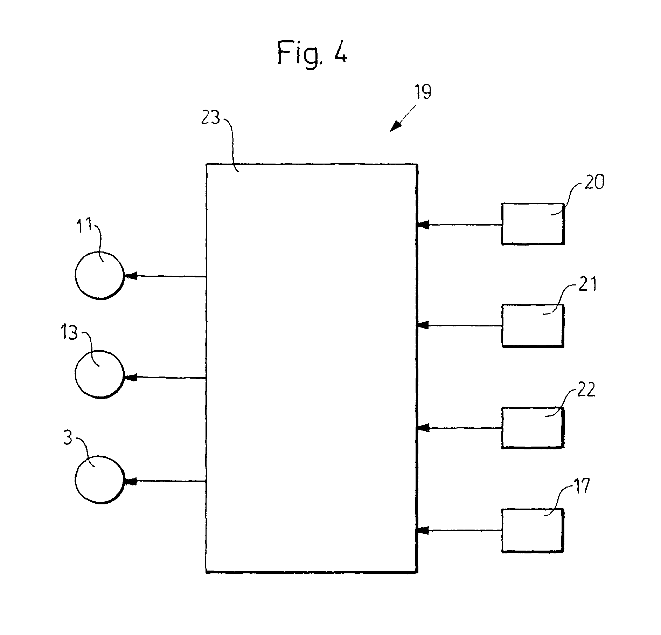

Die Lage der fiktiven Achse 18 wird mit Hilfe des Abstandsmesser 17 und einer später näher beschriebenen Steuervorrichtung, die in FIG. 4 mit 19 bezeichnet ist, ermittelt.

Das Drehen des Armes 6 um die fiktive Achse 18 erfolgt in einer gleichzeitigen Kombination sämtlicher Bewegungsmöglichkeiten der Armvorrichtung 2, d.h. teils durch das Drehen des Armes 6 um die Welle 10, teils durch eine Höhenverschiebung des Armes 6 und teils durch eine Seitenverschiebung des Armes 6.

In der FIG. 4 ist eine Steuervorrichtung 19 für die Armanordnung 2 in Form eines schematischen Blockschaltbildes gezeigt. Die Steuervorrichtung 19 besteht aus einem Lagesensor 20, der die Höhenlage des Armes 6 anzeigt, einem Lagesensor 21, der die Seitenlage des Armes anzeigt, sowie einem Drehmelder 22, der den Drehwinkel des Armes 6 angibt. Die hier erwähnten Sensoren 20, 21 bzw. der hier erwähnte Drehmelder 22 sind in keiner der gezeigten Figuren dargestellt. Sie können aber an strategischen Stellen am Mammographiegerät plaziert werden. Die Steuervorrichtung 19 besteht auch aus dem in Verbindung mit der FIG. 2 beschriebenen Abstandsmesser 17. Die Steuervorrichtung 19 weist weiterhin ein Steuerprogramm 23 auf, das beim Drehen des Armes 6 kontinuierlich Signale von den Sensoren 20, 21 bzw. vom Drehmelder 22, die den momentanen Höhen-, Seiten- und Drehpositionen des Armes 6 entsprechen, zugeführt bekommt. Das Steuerprogramm 23 hat auch Eingangssignale vom Abstandsmesser 17 gespeichert, die dem Abstand zwischen dem Objekttisch 8 und der Kompressionsplatte 9 entsprechen. Das Steuerprogramm 23 teilt nun den Wert des Eingangssignals durch zwei und rechnet damit die Lage der fiktiven horizontalen Achse 18, um die der Arm 6 gedreht werden soll, aus. Die fiktive horizontale Achse 18 liegt nun immer achsfluchtend mit der fiktiven Zentrumsachse der Brust, wenn sie sich in einer Kompressionslage befindet. Das Steuerprogramm 23 bearbeitet die zugeführte Information. Das Ergebnis der verarbeiteten Information wird nun dem Motor 11 zum Drehen des Armes 6, dem Motor 13 zum seitlichen Verschieben des Wagens 12 sowie dem Motor 3 zum vertikalen Verschieben des Armes 6 zugeführt. Eine Steuervorrichtung 19 der genannten Art ist durch die US-PS 4 019 059 bekannt und braucht daher nicht näher beschrieben zu werden. Lediglich der hier beschriebene Abstandsmesser 17 fehlt in Verbindung mit der Steuervorrichtung gemäss der US-Patentschrift.

Dadurch, dass die Steuervorrichtung 19 auf beschriebene Weise individuell für jeden Patienten eine optimale fiktive Achse 18, um die der Arm 6 gedreht werden kann, ausrechnen kann, ist unabhängig von der Größe der Brust eine Umpositionierung des Patienten von einer vertikalen Aufnahmestellung in eine gewünschte seitliche Aufnahmestellung nicht notwendig. Die fiktive Achse 18 ist immer entlang der fiktiven Längsachse 24 des Stativs 1, die parallel zum Schlitz 16 verläuft, angebracht.

## Patentansprüche

1. Röntgendiagnostikgerät für Mammographieuntersuchungen mit einem Arm (6) für eine Röntgenröhre (7) und einem Objekttisch (8) sowie einer zwischen der Röntgenröhre (7) und dem Objekttisch (8) angeordneten Kompressionsplatte (9), die mit dem Arm (6) verbunden und entlang diesem verschiebbar ist, wobei der Arm (6) um eine horizontale Welle (10) drehbar und entlang einer fiktiven Längsachse (24) eines Stativs (1) in der Höhe verschiebbar ist, wobei der Arm (6) mittels eines ersten Mittels (11) um die horizontale Welle (10) drehbar ist, dass die Welle (10) an einer Anordnung (12) befestigt ist, die mittels eines zweiten Mittels (13) seitlich verschiebbar ist, wodurch der Arm (6) seitlich verschiebbar ist, dass die Anordnung (12) zum seitlichen Verschieben des Armes (6) an einer mit dem Stativ (1) verbundenen Hubanordnung (14) verschiebbar angebracht ist, die mittels eines dritten Mittels (3) in der Höhe verschiebbar ist und dass eine Steuervorrichtung (19) vorhanden ist, die mittels eines Steuerprogramms, das Ausgangssignale von Lagesensoren (20, 21), die die Höhen- und Seitenlagen des Armes (6), sowie von einem Drehmelder (22), der den Drehwinkel des Armes (6) angibt, entgegennimmt, das zweite und das dritte Mittel (13, 3) für die Verschiebung des Armes (6) seitlich und in die Höhe sowie das erste Mittel (11) für die Drehung des Armes (6) derart steuert, dass eine Drehung des Armes (6) um eine entlang der fiktiven Längsachse (24) des Stativs (1) beliebig wählbare, fiktive Achse (18) ermöglicht wird, **dadurch gekennzeichnet, dass** die Steuervorrichtung (19) einen Abstandsmesser (17) aufweist, der an der Kompressionsplatte (9) angeordnet ist und durch den der Abstand der Kompressionsplatte (9) vom Objekttisch (8) messbar ist, so dass die fiktive horizontale Achse (18) achsfluchtend mit der fiktiven Zentrumsachse einer zu untersuchenden Brust gelegt ist, wenn sich diese in einer Kompressionslage befindet.

2. Röntgendiagnostikgerät nach Anspruch 1, **dadurch gekennzeichnet, dass** die fiktive Achse (18) mit einem Abstand vom Objekttisch (8) liegt, der dem halben Abstand zwischen dem Objekttisch (8) und der Kompressionsplatte (9) entspricht, wenn die Kompressionsplatte (9) sich in einer Kompressionslage befindet.

3. Röntgendiagnostikgerät nach Anspruch 1, **dadurch gekennzeichnet, dass** der Abstandsmesser (17) an dem Ende der Kompressionsplatte (9) angebracht ist, das gegen den Arm (6) gerichtet ist.

## Claims

1. X-ray diagnostic device for mammography examinations comprising an arm (6) for an X-ray tube (7) and a subject table (8) as well as a compression plate (9) arranged between the X-ray tube (7) and the subject table (8), said compression plate (9) being connected to said arm (6) and displaceable along it, wherein the arm (6) can be rotated around a horizontal shaft (10) and is height-adjustable along an imaginary longitudinal axis (24) of a stand (1), wherein the arm (6) is rotatable around the horizontal shaft (10) by means of a first means (11), the shaft (10) is secured to an arrangement (12) which is laterally displaceable by means of a second means (13), thereby making the arm (6) also laterally displaceable, the arrangement (12) for lateral displacement of the arm (6) is displaceably attached to a lifting arrangement (14) connected to the stand (1), said lifting arrangement (14) being height-adjustable by means of a third means (3), and a control device (19) is present which has a control programme that receives output signals both from position sensors (20, 21) which indicate the height position and lateral position of the arm (6) and from a synchro system (22) which indicates the rotational angle of the arm (6), said control programme controlling the second and the third means (13, 3) for displacement of the arm (6) laterally and vertically and also the first means (11) for rotating the arm (6) in such a way that a rotation of the arm (6) about an imaginary axis (18) that is arbitrarily selectable along the imaginary longitudinal axis (24) of the stand (1) is made possible,
**characterised in that** the control device (19) has a distance measuring unit (17) which is disposed on the compression plate (9) and by means of which the distance of the compression plate (9) from the subject table (8) can be measured so that the imaginary horizontal axis (18) lies in axial alignment with the imaginary central axis of a breast that is to be examined when the latter is in a compression position.

2. X-ray diagnostic device according to claim 1,
**characterised in that** the imaginary axis (18) lies at a distance from the subject table (8) that is equivalent to half the distance between the subject table (8) and the compression plate (9) when the compression plate (9) is in a compression position.

3. X-ray diagnostic device according to claim 1,
**characterised in that** the distance measuring unit (17) is attached to the end of the compression plate (9) which is directed towards the arm (6).

## Revendications

1. Appareil de radiodiagnostic pour des mammographies, comprenant un bras (6) pour un tube (7) radiogène et une tablette (8) objet, ainsi qu'une plaque (9) de compression disposée entre le tube (7) radiogène et la tablette (8) objet reliée au bras (6) et pouvant coulisser le long de celui-ci, le bras (6) pouvant tourner autour d'un arbre (10) horizontal et pouvant coulisser en hauteur le long d'un axe (24) longitudinal fictif d'un statif (1), le bras (6) pouvant tourner autour de l'arbre (10) horizontal à l'aide d'un premier moyen (11), en ce que l'arbre (10) est fixé à un équipage (12) qui peut coulisser latéralement à l'aide d'un deuxième moyen (13), le bras (6) pouvant coulisser latéralement, en ce que l'équipage (12) est monté coulissant pour le coulissement latéral de l'arbre (6) sur un dispositif (14) de levage relié au statif (1) et pouvant coulisser en hauteur à l'aide d'un troisième moyen (3), et en ce qu'il est prévu un dispositif (19) de commande qui, au moyen d'un programme de commande, reçoit les signaux de sortie de capteurs (20, 21) de position qui indiquent les positions en hauteur et latérale du bras (6), ainsi que d'un indicateur (22) de rotation qui indique l'angle de rotation du bras (6), commande le deuxième et le troisième moyen (13, 3) pour le coulissement de l'arbre (6) latéralement et en hauteur, ainsi que le premier moyen (11) pour la rotation du bras (6), de manière à permettre une rotation du bras (6) autour d'un axe (18) fictif pouvant être choisi à volonté le long de l'axe (24) longitudinal fictif du statif (1), **caractérisé en ce que** le dispositif (19) de commande a un dispositif (17) de mesure de distance qui est disposé sur la plaque (9) de compression et par lequel la distance entre la plaque (9) de compression et la tablette (8) objet peut être mesurée, de sorte que l'axe (18) horizontal fictif est mis en alignement avec l'axe central fictif d'une poitrine à étudier lorsque celle-ci se trouve en une position de compression.

2. Appareil de radiodiagnostic suivant la revendication 1, **caractérisé en ce que** l'axe (18) fictif est à une distance de la tablette (8) objet qui correspond à la moitié de la distance entre la tablette (8) objet et la plaque (9) de compression, lorsque la plaque (9) de compression se trouve dans une position de compression.

3. Appareil de radiodiagnostic suivant la revendication 1, **caractérisé en ce que** le dispositif (17) de mesure de distance est monté à l'extrémité de la plaque (9) de compression qui est dirigée vers le bras (6).
